# EUROPEAN PATENT APPLICATION

(11) **EP 3 964 225 A1**
(43) Date of publication of application: **09.03.2022**
(21) Application number: 20194685.2
(22) Date of filing: 04.09.2020
(51) Int. Cl.: A61K 38/16, A61P 9/10, A61P 15/06, A61P 17/02, A61P 25/28, C07K 14/16, A61K 38/18, C07K 14/155, C07K 14/505

(54) **PEPTIDES ENDOWED WITH ANGIOGENIC ACTIVITY**

(71) Applicant: Dompe' Farmaceutici S.P.A., 20122 Milan (IT)
(72) Inventor: Caruso, Arnaldo, 25123 Brescia (IT); Caccuri, Francesca, 25123 Brescia (IT)
(74) Representative: Mauri, Elisabetta Maria Ester

(57) **Abstract**

The present invention relates to a peptide, or derivative thereof, having length equal to or lower than 30 amino acids, and comprising any one of SEQ ID Nos. 2, 3, 9, 10, 13, 14, 15 or 16 or a conservative variant thereof, for use as a medicament, preferably for use in the treatment of conditions associated to/caused by deficient angiogenesis or conditions beneficing from increased angiogenesis.

SEQUENCE LISTING

<110> Dompé Farmaceutici SpA

<120> Peptide Endowed with Angiogenic Activity

<130> 235EP

<160> 16

<170> BiSSAP 1.3.6

<210> 1
<211> 20
<212> PRT
<213> Artificial Sequence

<220>
<223> F1

<400> 1
Gly Ala Arg Ala Ser Val Leu Ser Gly Gly Glu Leu Asp Arg Trp Glu
1               5                   10                  15
Lys Ile Arg Leu
            20

<210> 2
<211> 20
<212> PRT
<213> Artificial Sequence

<220>
<223> F2

<400> 2
Glu Lys Ile Arg Leu Arg Pro Gly Gly Lys Lys Lys Tyr Lys Leu Lys
1               5                   10                  15
His Ile Val Trp
            20

<210> 3
<211> 20
<212> PRT
<213> Artificial Sequence

<220>
<223> F3

<400> 3
Lys His Ile Val Trp Ala Ser Arg Glu Leu Glu Arg Phe Ala Val Asn
1               5                   10                  15
Pro Gly Leu Leu
            20

<210> 4
<211> 20
<212> PRT
<213> Artificial Sequence

<220>
<223> F4

<400> 4
Asn Pro Gly Leu Leu Glu Thr Ser Glu Gly Cys Arg Gln Ile Leu Gly
1               5                   10                  15
Gln Leu Gln Pro
            20

<210> 5
<211> 20
<212> PRT
<213> Artificial Sequence


<220>
<223> F5

<400> 5
Gly Gln Leu Gln Pro Ser Leu Gln Thr Gly Ser Glu Glu Leu Arg Ser
1               5                   10                  15
Leu Tyr Asn Thr
            20

<210> 6
<211> 20
<212> PRT
<213> Artificial Sequence


<220>
<223> F6

<400> 6
Ser Leu Tyr Asn Thr Val Ala Thr Leu Tyr Cys Val His Gln Arg Ile
1               5                   10                  15
Glu Ile Lys Asp
            20

<210> 7
<211> 20
<212> PRT
<213> Artificial Sequence


<220>
<223> F7

<400> 7
Ile Glu Ile Lys Asp Thr Lys Glu Ala Leu Asp Lys Ile Glu Glu Glu
1               5                   10                  15
Gln Asn Lys Ser
            20

<210> 8
<211> 36
<212> PRT
<213> Artificial Sequence


<220>
<223> F8

<400> 8
Thr Lys Glu Ala Leu Asp Lys Ile Glu Glu Glu Gln Asn Lys Ser Lys
1               5                   10                  15
Lys Lys Ala Gln Gln Ala Ala Ala Asp Thr Gly His Ser Ser Gln Val
            20                  25                  30
Ser Gln Asn Tyr
        35

<210> 9
<211> 16
<212> PRT
<213> Artificial Sequence

<220>
<223> F3S

<400> 9
Ala Ser Arg Glu Leu Glu Arg Phe Ala Val Asn Pro Gly Leu Leu Glu
1               5                   10                  15

<210> 10
<211> 11
<212> PRT
<213> Artificial Sequence

<220>
<223> EPO

<400> 10
Asp Ser Arg Val Leu Glu Arg Tyr Leu Leu Glu
1               5                   10

<210> 11
<211> 132
<212> PRT
<213> Human immunodeficiency virus type 1 BH10

<220>
<223> p17

<400> 11
Met Gly Ala Arg Ala Ser Val Leu Ser Gly Gly Glu Leu Asp Arg Trp
1               5                   10                  15
Glu Lys Ile Arg Leu Arg Pro Gly Gly Lys Lys Lys Tyr Lys Leu Lys
            20                  25                  30
His Ile Val Trp Ala Ser Arg Glu Leu Glu Arg Phe Ala Val Asn Pro
        35                  40                  45
Gly Leu Leu Glu Thr Ser Glu Gly Cys Arg Gln Ile Leu Gly Gln Leu
    50                  55                  60
Gln Pro Ser Leu Gln Thr Gly Ser Glu Glu Leu Arg Ser Leu Tyr Asn
65                  70                  75                  80
Thr Val Ala Thr Leu Tyr Cys Val His Gln Arg Ile Glu Ile Lys Asp
                85                  90                  95
Thr Lys Glu Ala Leu Asp Lys Ile Glu Glu Glu Gln Asn Lys Ser Lys
            100                 105                 110
Lys Lys Ala Gln Gln Ala Ala Ala Asp Thr Gly His Ser Ser Gln Val
        115                 120                 125
Ser Gln Asn Tyr

    130

<210> 12
<211> 207
<212> PRT
<213> Human immunodeficiency virus type 1 BH10

<220>
<223> p24

<400> 12
His Gln Ala Leu Ser Pro Arg Thr Leu Asn Ala Trp Val Lys Val Ile
1               5                   10                  15
Glu Glu Lys Ala Phe Ser Pro Glu Val Ile Pro Met Phe Ser Ala Leu
            20                  25                  30
Ser Glu Gly Ala Thr Pro Gln Asp Leu Asn Thr Met Leu Asn Thr Val
        35                  40                  45
Gly Gly His Gln Ala Ala Met Gln Met Leu Lys Asp Thr Ile Asn Glu
    50                  55                  60
Glu Ala Ala Glu Trp Asp Arg Met His Pro Val Gln Ala Gly Pro Ile
65                  70                  75                  80
Pro Pro Gly Gln Ile Arg Glu Pro Arg Gly Ser Asp Ile Ala Gly Thr
                85                  90                  95
Thr Ser Thr Leu Gln Glu Gln Ile Thr Trp Met Thr Ser Asn Pro Pro
            100                 105                 110
Ile Pro Val Gly Glu Ile Tyr Lys Arg Trp Ile Ile Leu Gly Leu Asn
        115                 120                 125
Lys Ile Val Arg Met Tyr Ser Pro Val Ser Ile Leu Asp Ile Arg Gln
    130                 135                 140
Gly Pro Lys Glu Pro Phe Arg Asp Tyr Val Asp Arg Phe Phe Arg Val
145                 150                 155                 160
Leu Arg Ala Glu Gln Ala Thr Gln Glu Val Lys Asn Trp Met Thr Glu
                165                 170                 175
Thr Leu Leu Val Gln Asn Ala Asn Pro Asp Cys Arg Thr Ile Leu Lys
            180                 185                 190
Ala Leu Gly Ser Gly Ala Thr Leu Glu Glu Met Met Thr Ala Cys
        195                 200                 205

<210> 13
<211> 11
<212> PRT
<213> Artificial Sequence

<220>
<223> None

<400> 13
Ala Ser Arg Glu Leu Glu Arg Phe Leu Leu Glu
1               5                   10

<210> 14
<211> 8
<212> PRT
<213> Artificial Sequence

<220>
<223> None

<400> 14
Ala Ser Arg Glu Leu Glu Arg Phe

1                 5

<210> 15
<211> 11
<212> PRT
<213> Artificial Sequence

<220>
<223> None

<400> 15
Ala Ala Asn Glu Leu Asp Arg Phe Leu Leu Glu
1               5                   10

<210> 16
<211> 8
<212> PRT
<213> Artificial Sequence

<220>
<223> None

<400> 16
Ala Ala Asn Glu Leu Asp Arg Phe
1               5

## Description

### FIELD OF THE INVENTION

The present invention relates to peptides endowed with angiogenic activity, thereby inducing proliferation, migration, and capillary-like tube formation in human endothelial cells (human ECs). The angiogenic peptides can be used for preventing and/or treating conditions associated to deficient angiogenesis or where induction of angiogenesis is beneficial.

### STATE OF THE ART

Angiogenesis plays a central role in various physiological processes within human body. Recent evidence has shown that a number of pathological conditions are caused by deficient angiogenesis or benefit from stimulation of angiogenesis. In these conditions, the administration of angiogenic active molecules has shown to be a valid therapeutic approach.

A number of studies have demonstrated that angiogenic active molecules are useful in the treatment of ulcers, in particular, gastric and duodenal ulcers, coronary artery diseases, ischemia, in particular cerebral and miocardial ischemia, cerebrovascular diseases and acute and chronic cutaneous wounds.

In these pathological conditions, the administration of molecules endowed with angiogenic activity improves tissue perfusion, delivers survival factors to sites of tissue repair, mobilize regenerative stem cell populations, and ultimately restore form and function to the tissue.

For example, in phase I clinical trials with basic Firboblast Growth Factor (bFGF), a potent angiogenic factor, in patients with gastric or duodenal ulcers that were refractory to conventional therapy was found to heal gastric ulcers that were caused by nonsteroidal antiinflammatory drugs (Hull et al, Gut 1995, (37) 5, 610-612,).

Angiogenic agents have also been shown to be useful in ischemic cardiovalscular disease, inducing cardiac tissue repair and regeneration. Although more than 2,000 patients with heart disease have received some form of experimental angiogenic therapy currently there are no FDA-approved angiogenic drugs to treat ischemic cardiovascular disease (Vale et al, Revista Espanola de Cardiologia, 2001, (54) 10: 1210-1224; Simons et al, Circulation, 2000, (102) 11: E73-E86).

Angiogenic active molecules may also find application in inducing bone regeneration, dental pulp regeneration, embryo implant and fetus persistence in pregnancy.

Vasculogenesis is a pivotal procedure during dental implant osseointegration as well as bone repair process.

For example, vascular endothelial growth factor (VEGF), a signal protein that stimulates angiogenesis and vasculogenesis, has been used in tissue regeneration and pulp regeneration experimental models favoring endodontic regenerative procedures (Mamatha Yadlapati, et al., J Endod., Volume 43, Number 1, Pages 77-83, January 2017). Also, VEGFhas been demonstrated to play a central role in bone repair (Mengkai Guang, et al., Journal of Oral Science, Vol. 59, No. 2, 215-223, 2017).

In addition, angiogenesis has been demonstrated to be important during pregnancy for both implantation and normal intrauterine development of the fetus.

For example, it has been demonstrated that angiogenesis induced by endometrial RAS plays a role around the time of embryo implantation, affecting subsequent pregnancy outcomes (Ruofan Qi, et al., Ther Adv Endocrinol Metab, 2020, Vol. 11: 1-12) and stimulation of angiogenesis and vasculogenesis by hCG, contributes to provide the placenta with an adequate maternal blood supply and optimal embryo nutrition during the invasion of the uterine endometrium (Gridelet V, et al., Front. Immunol., March 2020, Vol. 11, Article 343).

The human immunodeficiency virus type 1 (HIV-1) matrix protein variant p17 (p17) is a 132 amino acids (aa)-long structural multi-target protein endowed with different biologically activities and display, differently from the wild-type protein (refp17), B cell growth-promoting activity.

It plays a crucial role in both early and late stages of the virus life cycle.

The biological activity of p17 occurs after binding to heparan sulfate proteoglycans (De Francesco et al, J Biol Chem, 2011, 286: 19541-19548; Bugatti et al, J Biol Chem, 2013, 288: 1150-1161), to CXCR1 and CXCR2 (Caccuri et al PNAS, 2012, 109: 14580-14585; Giagulli et al, Blood, 2012, 119: 2274-2283), and to many other still unknown receptors (He et al, BBA Gen Sub, 2019, 1863: 13-24; Liu et al, Lab Invest, 2019, 99: 180-190).

Recently, p17 was found to exert a potent angiogenic and lymphangiogenic activity upon stress condition mediated by interaction with the CXCR1 and CXCR2 receptors (Caccuri et al, PNAS, 2012, 109: 14580-14585; Caccuri et al, ATVB, 2014; 34: 846-856). All p17 activities on human endothelial cells were sustained by activation of a secretory autophagy-based pathway (Mazzuca et al, J Virol, 2017, 91, pii: e00801-17).

In He W et al., Biochim Biophys Acta Gen Subj. 2019; 1863:13-24, in order to identify the epitopes of p17 responsible for interaction with receptors and B cell growth, eight peptides corresponding to 20 aminoacid sequences of p17 were tested for both their ability to bind to the p17 receptors and inducing B cell growth. Peptide F1, corresponding to aminoacids 2 to 21 of p17 (SEQ ID No. 1), was found to be the only one endowed with cell growth promoting activity, while Peptide F2, corresponding to aminoacids 17-36 of p17 (SEQ ID No. 2), was found capable to bind p17 receptors such as heparin and CXCR1 and CXCR2 receptors.

### SUMMARY OF THE INVENTION

The present inventors have now surprisingly found a number of different peptides derived from p17 which are capable of exerting angiogenic activity on endothelial cells.

More in particular, the inventors have surprisingly found that the peptides having SEQ ID No. 2 and SEQ ID No. 3, corresponding to Peptides F2 and F3 described in He W. et al. cited above, have a biological activity relevant for therapeutic purposes.

In details, as will be shown in the experimental section, these peptides are endowed with a potent angiogenic activity on human umbilical vein endothelial cells (HUVECs) and other endothelial cells of different origin, such aorta (HAECs), lung (HMVEC-Ls) and lymph node (LN-LECs).

Further, the inventors have also surprisingly found that peptides having SEQ ID No. 2 and SEQ ID No. 3 promote endothelial cells migration in a wound healing assay and have angiogenic activity either *ex vivo,* by using an aortic ring assay and *in vivo,* by using the chick chorioallantoic membrane (CAM) assay.

The inventors have also found that SEQ ID No. 2 and SEQ ID No. 3 angiogenic activity is mediated by different pathways.

More in particular, peptide having SEQ ID No. 2 exerts this activity by binding with chemokine receptors CXCR1 and CXCR2 and under stressed condition only, while peptide having SEQ ID No. 3 exerts its activity without interaction with CXCR1 and CXCR2 under both stressed and normal cell culture conditions.

Furthermore the angiogenic activity, involves an autophagy-based pathway only in case of peptide of SEQ ID No. 2, while activity of peptide of SEQ ID No. 3 is independent from autophagy.

By further analysis of the activity of the above peptides, the present inventors also identified further peptides, having sequences of SEQ ID No. 9; SEQ ID No. 13 SEQ ID No. 14; having a partial sequence of the peptide of SEQ ID No. 3 and maintaining a potent angiogenic activity.

Finally, sequence alignment between peptide of SEQ ID No. 3 and human erythropoietin (EPO) or Simian Immunodeficiency Virus (SIV) of chimpanzee (SIVcpz) allowed to identify an EPO-derived peptide of SEQ ID No. 10 and SIVcpz-derived peptides of SEQ ID No. 15 and SEQ ID No. 16, having sequence similarity to the peptide of SEQ ID No. 3, and also endowed with a potent angiogenic activity.

Accordingly, a first aspect of the present invention relates to a peptide, or derivative thereof, having length equal to or lower than 30 amino acids, and comprising any one of SEQ ID Nos. 2, 3, 9, 10, 13, 14, 15 or 16 or a conservative variant thereof, for use as a medicament.

Preferably, the above peptide for use in the treatment of conditions associated to/caused by deficient angiogenesis or conditions beneficing from increased angiogenesis.

A second aspect of the present invention relates to a pharmaceutical composition comprising (i) a peptide having length equal to or lower equal to or lower than 30 amino acids, and comprising any one of SEQ ID Nos. 2, 3, 9, 10, 13, 14, 15 or 16 or a derivative thereof, and (ii) at least one pharmaceutically acceptable ingredient.

A third aspect of the present invention relates to a therapeutic method for treating or preventing angiogenesis-related conditions in a subject in need thereof, comprising the systemic administration of a pharmaceutical composition comprising (i) a peptide having length equal to or lower than 30 amino acids, and comprising any one of SEQ ID Nos. 2, 3, 9, 10, 13, 14, 15 or 16 or derivative thereof, and (ii) at least one pharmaceutically acceptable ingredient.

A fourth aspect of the present invention relates to a polynucleotide having a sequence that codes for a peptide having length equal to or lower than 30 amino acids, and comprising any one of SEQ ID Nos. 2, 3, 9, 10, 13, 14, 15 or 16.

A fifth aspect of the present invention relates to a peptide having length equal to or lower than 30 amino acids, and comprising the SEQ ID No. 9, 10, 13, 14, 15 or 16.

### BRIEF DESCRIPTION OF THE FIGURES

The embodiments of the present invention will now be described, by way of example, with reference to the accompanying drawings, wherein:
Figure 1 shows the amino acid sequence of p17 protein as well as of the eight p-17 derived peptides F1 to F8,
Figure 2 shows the graphical representation of the results of the angiogenic activity assay on HUVECs as described in example 2,
Figure 3 shows the graphical representation of the results of the angiogenic activity assay on HAECs as described in example 2,
Figure 4 shows the graphical representation of the results of the angiogenic activity assay on HMVEC-Ls as described in example 2,
Figure 5 shows the graphical representation of the results of the angiogenic activity assay on LN-LECs as described in example 2,
Figure 6 shows the graphical representation of the results of the wound-healing assay on HUVECs as described in example 3,
Figure 7 shows the graphical representation of the results of the aortic ring assay as described in example 4,
Figure 8 shows the graphical representation of the results of the chick chorioallantoic membrane (CAM) assay as described in example 5,
Figure 9 shows the graphical representation of the results of the angiogenic activity assay on HUVECs with CXCR1 and CXCR2 inhibition as described in example 6,
Figure 10 shows the graphical representation of the results of the angiogenic activity assay on HUVECs under normal (A) and stressed (B) conditions as described in example 7,
Figure 11 shows the graphical representation of the results of the angiogenic activity assay promoted by EPO at various concentrations on HUVECs under normal (A) and stressed (B) conditions as described in example 8,
Figure 12 shows the graphical representation of the results of the angiogenic activity assay promoted by F3S and EPO peptide on HUVECs under normal (A) and stressed (B) conditions as described in example 8,
Figure 13 shows the graphical representation of the results of the angiogenic activity assay on HUVECs with the peptides ASRELERF (SEQ ID No 13) and ASRELERFLLE (SEQ ID No 14), conducted as described in example 2, and
Figure 14 shows the graphical representation of the results of the angiogenic activity assay on HUVECs with the peptides AANELDRF (SEQ ID No 16) and AANELDRFLLE (SEQ ID No 15), conducted as described in example 2.

### DETAILED DESCRIPTION OF THE INVENTION

In a first aspect, the invention relates to a peptide, or derivative thereof, having length equal to or lower than 30 amino acids, and comprising any one of SEQ ID Nos. 2, 3, 9, 10, 13, 14, 15 or 16 or a conservative variant thereof, for use as medicament.

Preferably, the above peptide is for use in the prevention or treatment in an individual of conditions associated to/caused by deficient angiogenesis or conditions beneficing from increased angiogenesis.

Peptides according to the several aspects of the present invention comprising the SEQ ID No. 2 have length preferably equal to or lower than 30 amino acids, more preferably equal to or lower than 25 amino acids, and still more preferably equal to or lower than 20 amino acids, even more preferably they have a sequence consisting of SEQ ID No. 2.

Peptides according to the several aspects of the present invention comprising the SEQ ID No. 3 have length preferably equal to or lower than 30 amino acids, more preferably equal to or lower than 25 amino acids, and still more preferably equal to or lower than 20 amino acids, even more preferably they have a sequence consisting of SEQ ID No. 3.

Peptides according to the several aspects of the present invention comprising the SEQ ID No. 9 have length preferably equal to or lower than 30 amino acids, more preferably equal to or lower than 25 amino acids, and still more preferably equal to or lower than 20 amino acids, even more preferably they have a sequence consisting of SEQ ID No 9.

Peptides according to the several aspects of the present invention comprising the SEQ ID No. 10 have length preferably equal to or lower than 30 amino acids, more preferably equal to or lower than 25 amino acids, and still more preferably equal to or lower than 20 amino acids, even more preferably they have a sequence consisting of SEQ ID No. 10.

Peptides according to the several aspects of the present invention comprising the SEQ ID No. 13 have length preferably equal to or lower than 30 amino acids, more preferably equal to or lower than 25 amino acids, and still more preferably equal to or lower than 20 amino acids, even more preferably they have a sequence consisting of SEQ ID No. 13.

Peptides according to the several aspects of the present invention comprising the SEQ ID No. 14 have length preferably equal to or lower than 30 amino acids, more preferably equal to or lower than 25 amino acids, and still more preferably equal to or lower than 20 amino acids, even more preferably they have a sequence consisting of SEQ ID No. 14.

Peptides according to the several aspects of the present invention comprising the SEQ ID No. 15 have length preferably equal to or lower than 30 amino acids, more preferably equal to or lower than 25 amino acids, and still more preferably equal to or lower than 20 amino acids, even more preferably they have a sequence consisting of SEQ ID No. 15.

Peptides according to the several aspects of the present invention comprising the SEQ ID No. 16 have length preferably equal to or lower than 30 amino acids, more preferably equal to or lower than 25 amino acids, and still more preferably equal to or lower than 20 amino acids, even more preferably they have a sequence consisting of SEQ ID No. 16.

Abbreviations of the amino acid sequences used herein are in accordance with the IUPAC-IUB nomenclature as reported in the following Table A.

**Table A**

| | | | | | |
|---|---|---|---|---|---|
| Alanine | Ala | A | Arginine | Arg | R |
| Asparagine | Asn | N | Aspartic acid | Asp | D |
| Cysteine | Cys | C | Glutamic acid | Glu | E |
| Glutamine | Gin | Q | Glycine | Gly | G |
| Histidine | His | H | Isoleucine | Ile | I |
| Leucine | Leu | L | Lysine | Lys | K |
| Methionine | Met | M | Phenylalanine | Phe | F |
| Proline | Pro | P | Serine | Ser | S |
| Threonine | Thr | T | Tryptophan | Trp | W |
| Tyrosine | Tyr | Y | Valine | Val | _{V} |

The term "conservative variant" as employed herein in relation to the aminoacid sequence of the peptides according to the invention, means a sequence that differs from said aminoacid sequence of the peptides for a conservative substitution of amino acids that do not influence the final peptide activity. The substitutions able to maintain the peptide activity are selected on the basis of (a) the efficacy in maintaining the structure of the peptide backbone in the area of substitution, such as sheet or helical three-dimensional structures, (b) the efficacy in maintaining electrical charge or hydrophobicity of the molecule in the target area, or (c) the efficacy of maintaining the bulk of the side chain.

Amino acids are classified according to general side chain properties as described in the following Table B.

**Table B**

| | |
|---|---|
| Hydrophobicity | NorLeucine, Met, Ala, Val, Leu, Ile; |
| neutral hydrophobicity | Cys, Ser, Thr; |
| Acidity | Asp, Glu; |
| Basicity | Asn, Gln, His, Lys, Arg; |
| residue that affects chain orientation | Gly, Pro; |
| Aromaticity | Trp, Tyr, Phe. |

Examples of conservative substitution belong to the group consisting of basic amino acids (arginine, lysine and histidine), acidic amino acids (glutamic acid and aspartic add), polar amino acids (glutamine and asparagine), hydrophobic amino acids (leucine, isoleucine, valine and methionine), aromatic amino acids (phenylalanine, tryptophan and tyrosine), and small amino acids (glycine, alanine, serine, and threonine).

The amino acid substitutions that do not generally alter the specific activity are known in the art of the present invention.

Most common occurred alteration are Ala/Ser, Val/lle, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly, and the opposite alterations. Another example of conservative substitutions are shown in the following Table C.

**TABLE C**

| Starting amino acid | Possible substitution | Preferred substitution |
|---|---|---|
| Ala (A) | Val; Leu; lie | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gin | Asp |
| Gly (G) | Ala | Ala |
| His (H) | Asn; Gln; Lys; Arg | Arg |
| lie (I) | Leu; Val; Met; Ala; Phe; norLeucine | Leu |
| Leu (L) | norLeucine; Ile; Val; Met; Ala; Phe | lie |
| Lys (K) | Arg; Gln; Asn | Arg |
| Met (M) | Leu; Phe; Ile | Leu |
| Phe (F) | Leu; Val; Ile; Ala; Tyr | Tyr |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala; norLeucine | Leu |

The peptide according to the several aspects of the present invention may be in the form of a modified peptide, wherein the N- or/and C-terminal thereof is chemically modified or protected with organic compounds.

The term "derivative" or "derivative thereof" as employed herein in relation to a peptide according to the several aspects of the present invention means a peptide wherein the N- and/or C-terminal thereof is chemically modified or protected with an organic compound.

Examples of modification include phosphorylation, glycosylation, acylation (including acetylation, lauroylation, myristorylation, palmitoylation), alkylation, carboxylation, hydroxylation, glycation, biotinylatlon, ubiquitinylation, and amidation.

Preferably, the peptide according to the several aspects of the present invention may be modified at the N-terminal thereof, more preferably by acylation, including acetylation, lauroylation, myristorylation, and palmitoylation. N-terminal acetyl and palmitoyl peptide derivatives are a preferred aspect of the present invention.

Depending on its length, the peptide according to the several aspects of the present invention may be synthesized by a method well known in the art, for example, by an automated peptide synthesizer, or produced by a genetic engineering technology. For example, a fusion gene encoding a fusion protein including a fusion partner and the peptide is prepared by genetic engineering, and then transformed into a host cell to express the fusion protein. Thereafter, the peptide is cleaved and isolated from the fusion protein using a protease or a compound so as to produce the desired peptide. To this end, a DNA sequence encoding amino acid residues which can be cleaved by a protease such as Factor Xa or enterokinase, or a compound such as CNBr or hydroxylamine may be inserted between the polynucleotides encoding the fusion partner and the peptide.

The peptides according to the several aspects of the present invention may exist as stereoisomers or mixtures of stereoisomers; for example, the amino acids that make them up can have L-configuration, D-configuration or be racemic independently from each other. Therefore, it is possible to obtain isomeric mixtures as well as racemates or diastereomeric mixtures or pure diastereomers or enantiomers, depending on the number of asymmetric carbons and what isomers or isomeric mixtures are present. The preferred structures of the peptides are pure isomers, i.e., enantiomers or diastereomers. The most preferred structures of the peptides include amino acids having the L-configuration. Unless otherwise indicated, it is understood that when it is indicated that one amino acid can be Ala, it is understood that it is selected from L-Ala-, D-Ala- or racemic or non-racemic mixtures of both.

As used herein, the term, "angiogenesis" refers to the growth of new blood vessels, or "neovascularization," and involves the growth of new blood vessels of relatively small caliber composed of endothelial cells. Angiogenesis is an integral part of many important biological processes including cancer cell proliferation, solid tumor formation, inflammation, wound healing, repair of injured ischemic tissue, myocardial revascularization and remodeling, ovarian follicle maturation, menstrual cycle, and fetal development. New blood vessel formation is required for the development of any new tissue, whether normal or pathological, and thus represents a potential control point in regulating many disease states, as well as a therapeutic opportunity to encourage growth of normal tissue and "normal" angiogenesis.

According to a preferred embodiment of the first aspect of the invention, the above peptide is for use in the treatment of ischemic diseases, arterial diseases, cerebrovascular diseases, ulcers, acute and chronic cutaneous wounds, bone and dental pulp loss and in the prevention of spontaneous abortion.

Said bone and dental loss may be subsequent to diseases or trauma.

The peptides described herein induce angiogenic proliferation and migration of endothelial cells resulting in formation of new capillaries and collateral vessels to promote healing of wounds and ulcers, to restore function to damaged cardiovascular tissue in ischemic, arterial or cerebrovascular diseases, to induce bone or dental pulp regeneration in case of bone or dental tissue loss and to support embryo implanationt and persistence during pregnancy.

Ulcers and cutaneous wounds are injuries or lesions caused by physical means, such as mechanical, chemical, bacterial, or thermal means, which disrupt the normal continuity of structures of the internal mucosae or of the skin, respectively.

Said ulcers are preferably selected from gastic and duodenal ulcers.

Said ischemic diseases are preferably selected from ischemic heart disease, myocardial infarction, acute myocardial infarction, myocardosis, cardiomyopathy, angina pectoris, unstable angina.

Repair of injured tissue due to ischemia requires extensive remodeling and revascularization. An infarction is, by definition, an area of tissue ischemic necrosis caused by occlusion of local blood circulation. The resulting necrotic lesion leaves the affected tissue deprived of oxygen and nutrients. In the heart, obstruction of coronary circulation in particular, results in myocardial infarction. As the ischemic myocardium undergoes rapid oxygen starvation, the hypoxic microenvironment of the affected cardiac muscle induces the synthesis of angiogenic factors to attempt re-vascularization. In this situation, the provision of angiogenic factors is useful in order to support the re-vascularization of the tissue.

Said arterial diseases are preferably selected from coronary arteriosclerosis, heart failure, arteriosclerosis obliterans (ASO), Berger's disease, vascular injury, arterial embolism, arterial thrombosis, arterial occlusion of the organ, and aneurysm.

Said cerebrovascular diseases are preferably selected from cerebrovascular occlusion, cerebral infarction, cerebral thrombosis, cerebral embolism, stroke, cerebral hemorrhage, moyamoya disease, cerebrovascular dementia, Alzheimer type dementia, sequela of cerebral hemorrhage, and sequela of cerebral infarction.

Preferably, said bone and dental pulp loss is a consequence of a trauma or disease. Regeneration of the lost tissue is particularly important to properly restore the lost tissue. The administration of the peptides of the invention is particularly useful in maxillofacial surgery, bone grafts, prostheses (hip, knee, ear, etc.) and in dental implants.

The administration of molecules that improve vasularization of the uterus and thus assist embryo implantation and persistence is essential to promote pregnancy and the prosecution of pregnancy and prevent spontaneous abortion in women with uterine vascularisation defects. Preferably, the peptide of the invention is for use in the prevention of recurrent spontaneous abortion.

According to a further preferred embodiment of the first aspect of the invention, the above peptide is also for use as an adjuvant therapy in assisted reproduction technology.

In accordance with this embodiment, the peptide is administered to a woman undergoing assisted reproduction technology in order to facilitate embryo implantation and/or development.

A second aspect the present invention relates to a pharmaceutical composition comprising (i) a peptide as above described according to the first aspect of the invention, and (ii) at least one pharmaceutically acceptable ingredient.

A third aspect of the present invention relates to a therapeutic method for treating conditions associated to/caused by deficient angiogenesis or beneficing from increased angiogenesis as defined above, in a subject in need thereof, comprising the systemic administration of the above described pharmaceutical composition.

The pharmaceutical composition of the present invention can comprise an amount of the peptide, or a derivative and/or salt thereof, ranging from 0.00000001 % to 20% by weight, preferably from 0.000001 % to 15% by weight, more preferably from 0.0001 % to 10% by weight, and even more preferably from 0.0001 % to 5% by weight.

Preferably, the pharmaceutical composition of the present invention is prepared in suitable dosage forms comprising an effective amount of at least one of the above described peptides together with at least one pharmaceutically acceptable ingredient.

Examples of suitable dosage forms are tablets, capsules, coated tablets, granules, solutions and syrups for oral administration; solutions, pomade and ointment for topical administration; medicated patches for transdermal administration; suppositories for rectal administration and injectable sterile solutions. Other suitable dosage forms are those with sustained release and those based on liposomes for oral, injectable or transdermal administration.

As described herein, the pharmaceutical composition of the present invention comprises at least one of the above described peptides together with a pharmaceutically acceptable excipient, which, as used herein, includes any and all solvents, diluents, or other vehicle, dispersion or suspension aids, surface active agents, isotonic agents, thickening or emulsifying agents, preservatives, solid binders, lubricants and the like, as suited to the particular dosage form desired.

Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, other non-toxic compatible lubricants such as sodium lauryl sulfate and magnesium stearate, coloring agents, releasing agents, coating agents, sweetening, flavoring and perfuming agents, preservatives and antioxidants.

The terms "pharmaceutically acceptable" and "physiologically acceptable" are intended to define, without any particular limitation, any material suitable for preparing a pharmaceutical composition to be administered to a living being.

The dosage forms can also contain other traditional ingredients such as: preservatives, stabilizers, surfactants, buffers, salts for regulating osmotic pressure, emulsifiers, sweeteners, colorants, flavourings and the like.

The pharmaceutical compositions of this invention may be administered orally, parenterally, by inhalation spray, topically, rectally, nasally, buccally, vaginally or via an implanted reservoir. The term parenteral as used herein includes subcutaneous, intracutaneous, intravenous, intramuscular, intraarticular, intrasynovial, intrasternal, intrathecal, intralesional and intracranial injection or infusion techniques.

The pharmaceutical compositions of this invention may also be administered by nasal aerosol or inhalation or delivered by implantation (e.g., surgically), such as with an implantable or indwelling device like a stent.

The dosage forms of the pharmaceutical composition of the present invention can be prepared by techniques that are familiar to a pharmaceutical chemist, and comprise mixing, granulation, compression, dissolution, sterilization and the like.

A fourth aspect of the present invention relates to a polynucleotide having a sequence that codes for a peptide according to the first aspect of the invention.

Preferably, said polyncleotide codes for a peptide having a sequence consisting of any one of SEQ ID Nos. 2, 3, 9, 10, 13, 14, 15 or 16 or a conservative variant thereof, more preferably SEQ ID No. 9, 10, 13, 14, 15 or 16.

A polynucleotide is a nucleic acid molecule that can be spontaneous or artificial DNA or RNA molecules, either single-stranded or double-stranded. The nucleic acid molecule can be one or more nucleic acids of same type (for example, having a same nucleotide sequence) or nucleic acids of different types. The nucleic acid molecules comprise one or more DNA, cDNA, decoy DNA, RNA, siRNA, miRNA shRNA, stRNA, snoRNA, snRNA PNA, antisense oligomer, plasmid and other modified nucleic acids, but not limited to those.

The above polynucleotide mentioned can be used in a method for the recombinant production of the peptides according to the invention.

A fifth aspect of the present invention relates to a peptide having length equal to or lower than 30 amino acids, and comprising the SEQ ID No. 9, 10, 13, 14, 15 or 16 or a conservative variant thereof.

Preferably, said peptide has a sequence that consists of SEQ ID No. 9, 10, 13, 14, 15 or 16 or a conservative variant thereof.

The invention will be further described in the following examples, which do not limit the scope of the invention described in the claims.

### EXPERIMENTAL PART

### Example 1 - Starting materials

### Recombinant proteins and peptides

Purified endotoxin (lipopolysaccharide)-free recombinant p17 (from clone BH10 of clade B isolate - SEQ ID No. 11), GST, and p24 (SEQ ID No. 12) were produced as described by De Francesco et al, Proc. Natl. Acad. Sci. USA. 2002; 99:9972-9977. The absence of endotoxin contamination (<0.25 endotoxin units/ml) in protein preparations was assessed by the Limulus amebocyte assay (Associates of Cape Cod, Inc.).

The nine p17-derived peptides F1-F8 (SEQ ID No. 1-8) and F3S (SEQ ID No. 9) and the EPO peptide (SEQ ID No. 10) were synthetized according to the conventional methods, known in the art, such as solid phase peptide synthesis methods, enzymatic synthesis or any combination (Bondazky et al., Int. J. Pept. Protein Res. (1993), 42(1):10-3).

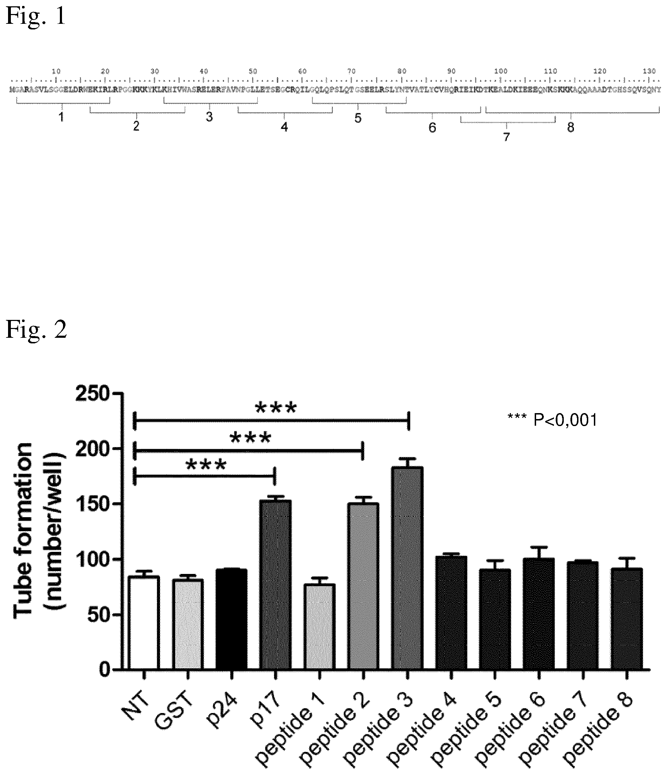

The first 7 peptides (F1-F7) each comprises 20 amino acids with 5 overlapping residues, and the C-terminal peptide (F8) contains 36 amino acids with 15 overlapping residues (**Figure 1**). Peptide F8 comprises 16 amino acids, 15 thereof overlapping peptide F3. EPO peptide comprises 11 amino acids.

### Cell Cultures

Human umbilical vein endothelial cells (HUVECs) were isolated and characterized as described in Caruso et al, Proc. Natl. Acad. Sci. USA. 2009; 106:20446-20451, and cultured in endothelial growth medium (EGM) (Lonza, Milan, Italy) containing 10% (vol/vol) fetal bovine serum (FBS).

Human aortic endothelial cells (HAECs) and Human lung microvascular endothelial cells (HMVEC-Ls) were purchased from Clonetics (San Diego, CA, USA) and cultured in EGM-2MV (Lonza) containing 10% (vol/vol) FBS.

Human primary lymph node-derived endothelial cells (LN-LECs) have been developed and characterized as described in Norder et al, FASEB J, 2012; 26:2835-2846.

LN-LECs were cultured in EGM (Lonza) containing 10% FBS and supplemented with VEGF-C (25 ng/ml) (Reliatech, Wolfenbuettel, Germany).

All experiments were carried out with cells at passages 2-6. All data were analyzed for statistical significance using one-way analysis of variance (ANOVA). Bonferroni's post-test was used to compare data. Differences were considered significant at a P value of <0.05. Statistical tests were performed using Prism 5 software (GraphPad).

### Example 2 - Angiogenic Activity Assay

Low passage HUVECs were grown under stressed (serum starved; 0.5% FBS) conditions for 16 h in endothelial basal medium (EBM) and then harvested and resuspended in complete medium containing 10% FBS.

Cells were seeded (5 x 10⁴ cells per well) in wells coated with growth factor-reduced Cultrex basement membrane extract (BME; Trevigen Gaithersburg, MD, USA) and left untreated (NT) or treated with 10 ng/ml of GST, p24, p17 or each p17-derived peptide F1 to F8.

Data are the means ± SD of three independent experiments with similar results. Statistical analysis was performed by one-way ANOVA and the Bonferroni post-test was used to compare data (***P<0.001).

Activity screening at a single concentration of 10 ng/ml identified two different peptides out of the eight, namely peptide F2 (SEQ ID No. 2) and F3 (SEQ ID No. 3), endowed with angiogenic activity (**Figure 2**).

The angiogenic activity of the two peptides was found to be similar to that exerted by p17. As expected the negative controls glutathione S-transferase (GST) and the HIV-1 capsid protein p24 (p24), did not induce any pro-angiogenic activity on HUVECs.

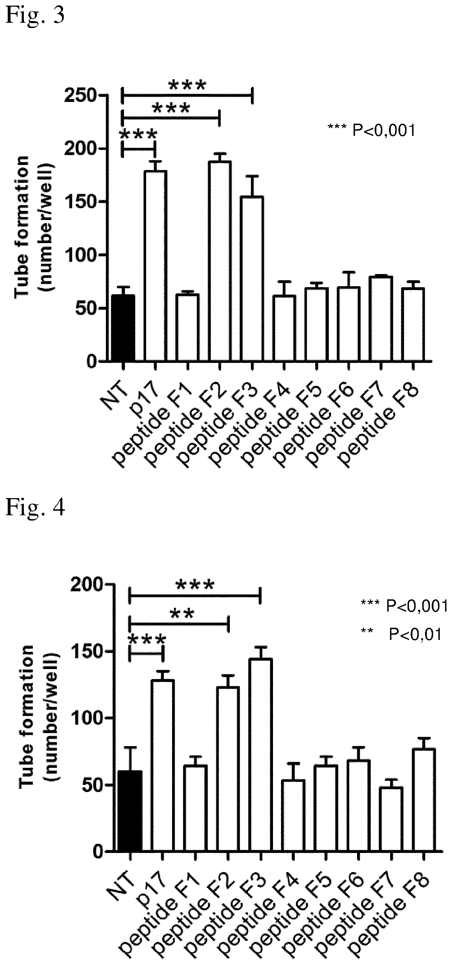

Similar results were obtained by using human ECs derived from different origin, such as aorta (HAECs), lung (HMVEC-Ls) and lymph node (LN-LECs) (**Figures 3, 4** **and** **5****,** respectively).

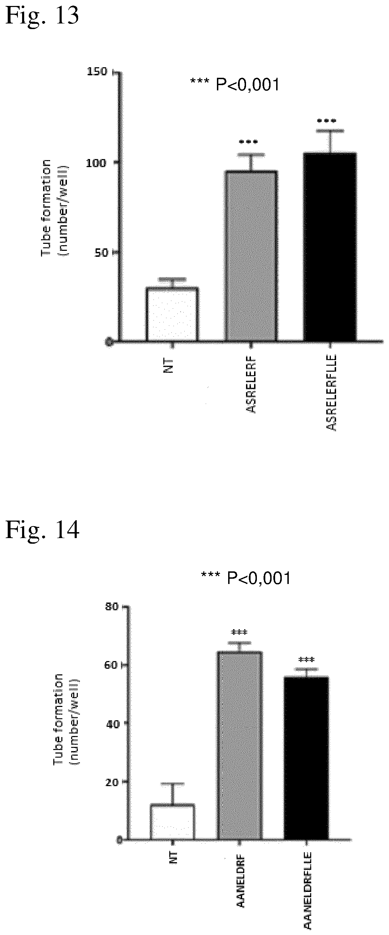

The same experiment was conducted on HUVEC cells with 10 ng of the peptides ASRELERF (SEQ ID No 13) and ASRELERFLLE (SEQ ID No 14) or the peptides AANELDRF (SEQ ID No 16) and AANELDRFLLE (SEQ ID No 15). The results are shown in **Figure 13** and **Figure 14****,** respectively, and show that all these peptides were able to significantly promote angiogenesis on HUVECs.

### Example 3 - Wound-healing Assay

The involvement of peptide F2 and F3 in promoting EC migration was evaluated by perfomring a wound healing assay.

HUVECs were plated on 24-well plates (10⁵ cells per well) in complete medium containing 10% FBS. Confluent monolayers were nutrient starved for 16 h and then scratched using a 200 µl pipette tip. After being washed, cells were left untreated or treated with 10 ng/ml of GST, p24, p17, or each p17-derived peptide F1 to F8.

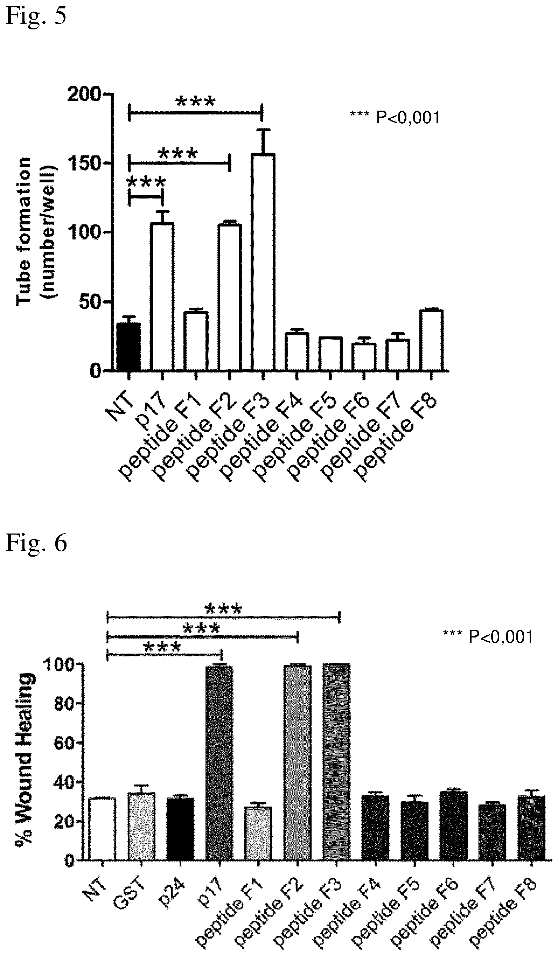

As shown in **Figure 6****,** not treated (NT) HUVECs reached approximately 31,73% (standard deviation [SD], ± 1%) healing after 12 h of culture, whereas at the same time, HUVECs treated with either peptide F2 or F3 showed almost 100% of healing. Activity of peptide F2 and F3 was comparable to that exerted by p17. As expected, GST and p24 used as negative controls did not exert any effect on HUVEC migration.

### Example 4 - Aortic ring assay

The vasculogenic activity of peptide F2 and F3 was further characterized by *ex vivo* experiments by using the rat aortic ring assay as described in Nicosia RF et al, Lab Invest, 1990; 63:115-122.

Briefly, the dorsal aorta was excised from 6-wk-old Sprague-Dawley rats. Rings, 1-mm thick, were embedded in collagen gel and then incubated with serum-free EBM containing PBS, peptide F2 (10 ng/ml), peptide F3 (10 ng/ml), or EGF-2 (50 ng/ml) (Thermo Fisher Scientific, Rodano, Italy). The plates were incubated for 10 days and angiogenesis was quantified by counting the number of microvessels originating from aortic rings.

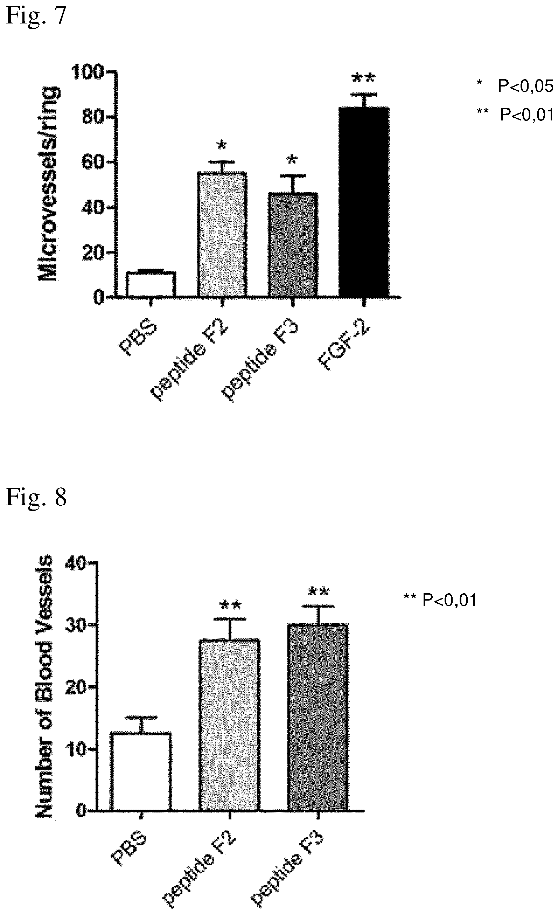

As shown in **Figure 7**, the number of microvessels in not treated (PBS) cultures, used as negative control, was significantly lower (11 ± 1) than that of rings treated with peptide F2 (55 ± 5) or peptide F3 (46 ± 8). As expected, stimulation of aortic rings with 50 ng/ml of FGF-2 strongly increased microvessel outgrowth (84 ± 6).

### Example 5 - Chick chorioallantoic membrane (CAM) assay

The vasculogenic activity of peptide F2 and F3 was further characterized by *in vivo* experiments by using the chick chorioallantoic membrane (CAM) assay.

Fertilized White Leghorn chicken eggs (30 per group) were incubated at 37°C at constant humidity. On day 3, a square window was opened in the shell, and 2-3 ml of albumin was removed to allow detachment of the developing CAM. The window was sealed with a glass, and the eggs were returned to the incubator. On day 8, eggs were treated with 1 mm³ sterilized gelatin sponges (Gelfoam; Upjohn, Kalamazoo, MI, USA) placed on top of the growing CAM, as described in Ribatti D et al, Nat Protoc, 2006; 1:85-91, and loaded with 1 µl of PBS (negative control), 1 µl of PBS containing 100 ng of peptide F2 or F3. CAMs were examined daily until day 12 and photographed in ovo with a stereomicroscope equipped with a camera and image analyzer system (Olympus, Hamburg, Germany). At day 12, the angiogenic response was evaluated by the image analyzer system and counted as the number of vessels converging toward the sponge.

As shown in **Figure 8****,** a significant angiogenic response in the form of numerous allantoic neovessels developing radially toward the implant in a "spoke-wheel" was promoted by peptide F2 and F3 (mean number of vessels: 27 ± 4 and 30 ± 3, respectively) as compared to PBS (mean number of vessels of 12 ± 2).

### Example 6 - Angiogenic Activity Assay with CXCR1 and CXCR2 Inhibiion

Low passage HUVECs were grown under stressed (serum starved; 0.5% FBS) conditions for 16 h in endothelial basal medium (EBM) and then harvested and resuspended in complete medium containing 10% FBS.

Cells were seeded (5 x 10⁴ cells per well) in wells coated with growth factor-reduced Cultrex basement membrane extract (BME; Trevigen Gaithersburg, MD, USA) and left untreated (NT) or treated with 10 ng/ml of each p17-derived peptide F1 or F2, after preincubation for 1 hour at 37°C with 2.5 µg/ml of a control isotype-matched mAb (Ctrl mAb), a neutralizing mAb to CXCR1 and/or a neutralizing mAb to CXCR2.

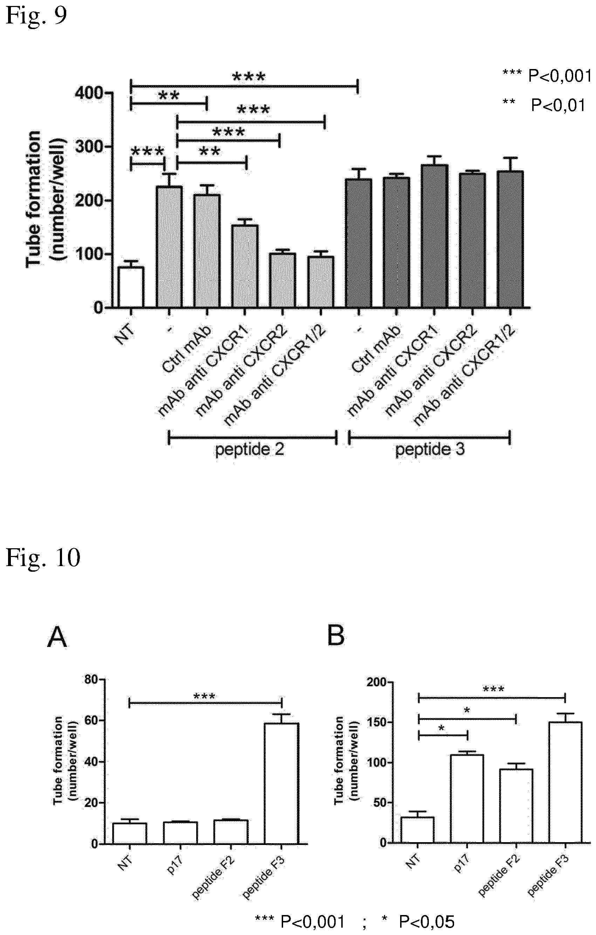

As shown in **Figure 9****,** the neutralizing mAb to CXCR1 and CXCR2 were inhibitory toward peptide F2-induced angiogenesis. On the contrary, CXCR1 and CXCR2 neutralizing mAbs did not impair the angiogenic activity of peptide F3.

These results suggested that peptide F3 does not use CXCR1 and CXCR2 to exert its angiogenic activity.

### Example 7 - Angiogenic Activity Assay under Normal and Stressed Condition

Low passage HUVECs were grown under normal (10% FBS) or stressed (serum starved; 0.5% FBS) conditions for 16 h in endothelial basal medium (EBM) and then harvested and resuspended in complete medium containing 10% FBS. Cells were seeded (5 x 10⁴ cells per well) in wells coated with growth factor-reduced Cultrex basement membrane extract (BME; Trevigen Gaithersburg, MD, USA) and left untreated or treated with 10 ng/ml of p17, F2 or F3.

As shown in **Figure 10A****,** under normal culture conditions, peptide F3 was found to promote angiogenesis as compared to not treated cells or to cells treated with peptide F2 or p17. As expected, HUVECs cultured under serum-deprived conditions were highly susceptible to stimulation with p17 as compared to control cells. Under stressed conditions, both peptides F2 and F3 were found to exert a potent angiogenic activity as compared to control, not treated cells (**Figure 10B**).

These results, together with those of example 6, suggested that peptide F2 acts as p17 by binding to CXCR1 and CXCR2 and promoting angiogenesis under stress condition only, while peptide F3 promotes angiogenesis under both stressed and normal cell culture condition, without interacting with CXCR1 and CXCR2, but possibly with a still unknown receptor.

### Example 8 - Angiogenic Activity Assay - Mimicry with EPO

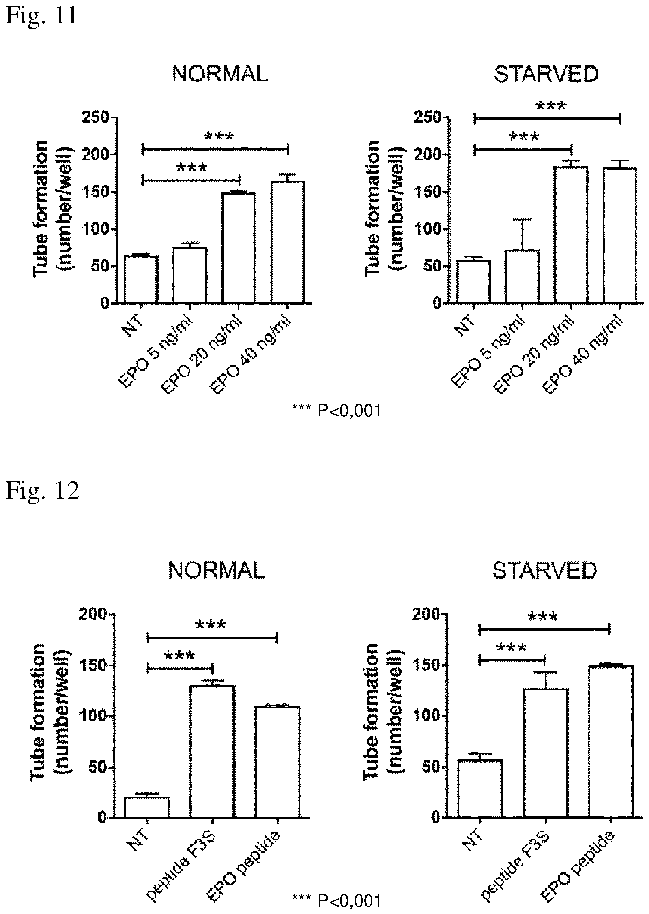

Human erythropoietin (EPO) is endowed of angiogenic activity on human ECs even if the epitope involved in such activity is unknown up-to-date. The procedure of example 7 was repeated by leaving the cells untreated or treated with 5, 20 or 40 ng/ml of EPO, showing that EPO significantly promoted angiogenesis at a concentration of 20 ng/ml under both normal (A) or starved (B) culture conditions (**Figure 11**).

Sequence alignment between peptide F3 and EPO allowed to identify the best matching in the EPO N-terminal region included between amino acid 8 to 10.

An EPO-derived 11 amino acids-long peptide (EPO peptide - SEQ ID No. 10) and a F3-derived 16 amino acids-long peptide (F3S - SEQ ID No. 9) were synthetized and tested at 10 ng/ml for their angiogenic activity under the same procedure of Example 7.

As shown in **Figure 12****,** EPO peptide exerted a potent angiogenic activity, comparable to that of F3-derived 16 amino acids-long peptide, under both normal (A) and stressed (B) culture conditions.

### SEQUENCE LISTING

SEQ ID No. 1
   GARASVLSGGELDRWEKIRL
SEQ ID No. 2
   EKIRLRPGGKKKYKLKHIVW
SEQ ID No. 3
   KHIVWASRELERFAVNPGLL
SEQ ID No. 4
   NPGLLETSEGCRQILGQLQP
SEQ ID No. 5
   GQLQPSLQTGSEELRSLYNT
SEQ ID No. 6
   SLYNTVATLYCVHQRIEIKD
SEQ ID No. 7
   IEIKDTKEALDKIEEEQNKS
SEQ ID No. 8
   TKEALDKIEEEQNKSKKKAQQAAADTGHSSQVSQNY
SEQ ID No. 9
   ASRELERFAVNPGLLE
SEQ ID No. 10
   DSRVLERYLLE 
SEQ ID No. 11

MGARASVLSGGELDRWEKIRLRPGGKKKYKLKHIVWASRELERFAVNPGLLETSEGC
RQILGQLQPSLQTGSEELRSLYNTVATLYCVHQRIEIKDTKEALDKIEEEQNKSKKKAQ
QAAADTGHSSQVSQNY
 
SEQ ID No. 12

HQALSPRTLNAWVKVIEEKAFSPEVIPMFSALSEGATPQDLNTMLNTVGGHQAAMQM
LKDTINEEAAEWDRMHPVQAGPIPPGQIREPRGSDIAGTTSTLQEQITWMTSNPPIPVG
EIYKRWIILGLNKIVRMYSPVSILDIRQGPKEPFRDYVDRFFRVLRAEQATQEVKNWMT
ETLLVQNANPDCRTILKALGSGATLEEMMTAC
SEQ ID No 13
   ASRELERFLLE
SEQ ID No. 14
   ASRELERF
SEQ ID No. 15
   AANELDRFLLE
SEQ ID No. 16
   AANELDRF

## Claims

1. A peptide, or derivative thereof, having length equal to or lower than 30 amino acids, and comprising any one of SEQ ID Nos. 2, 3, 9, 10, 13, 14, 15 or 16 or a conservative variant thereof, for use as a medicament.

2. A peptide as claimed in claim 1, for use in the prevention or treatment of conditions associated to/caused by deficient angiogenesis or conditions beneficing from increased angiogenesis.

3. A peptide for use as claimed in claims 1 or 2, having sequence consisting of SEQ ID Nos. 2, 3, 9, 10, 13, 14, 15 or 16 or a conservative variant thereof.

4. The peptide for use according to claim 1 to 3, for use in the treatment of ischemic diseases, arterial diseases, cerebrovascular diseases, ulcers, acute and chronic cutaneous wounds, bone and dental pulp loss and in the prevention of spontaneous abortion.

5. The peptide for use according to claims 1 to 4, wherein said ulcers are preferably selected from gastic and duodenal ulcers.

6. The peptide for use according to claims 1 to 4, wherein said ischemic diseases are preferably selected from ischemic heart disease, myocardial infarction, acute myocardial infarction, myocardosis, cardiomyopathy, angina pectoris and unstable angina.

7. The peptide for use according to claims 1 to 4, wherein said arterial diseases are preferably selected from coronary arteriosclerosis, heart failure, arteriosclerosis obliterans (ASO), Berger's disease, vascular injury, arterial embolism, arterial thrombosis, arterial occlusion of the organ, and aneurysm.

8. The peptide for use according to claims 1 to 4, wherein said cerebrovascular diseases are preferably selected from cerebrovascular occlusion, cerebral infarction, cerebral thrombosis, cerebral embolism, stroke, cerebral hemorrhage, moyamoya disease, cerebrovascular dementia, Alzheimer type dementia, sequela of cerebral hemorrhage, and sequela of cerebral infarction.

9. The peptide for use according to claims 1 to 4, wherein said spontaneous abortion is recurrent spontaneous abortion.

10. The peptide for use according to claim 1 to 3, for use as adjuvant therapy in assisted reproduction technology.

11. A pharmaceutical composition comprising (i) a peptide, or derivative thereof, having length equal to or lower than 30 amino acids, and comprising any one of SEQ ID Nos. 2, 3, 9, 10, 13, 14, 15 or 16 or a conservative variant thereof, and (ii) at least one pharmaceutically acceptable excipient.

12. A polynucleotide that codes for a peptide, or derivative thereof, having length equal to or lower than 30 amino acids, and comprising any one of SEQ ID Nos. 2, 3, 9, 10, 13, 14, 15 or 16 or a conservative variant thereof.

13. A peptide having length equal to or lower than 30 amino acids, and comprising the SEQ ID No. 9, 10, 13, 14, 15 or 16.

14. A peptide having a sequence consisting of SEQ ID No. 9, 10, 13, 14, 15 or 16.
